# EUROPEAN PATENT SPECIFICATION

(11) **EP 2 918 759 B1**
(45) Date of publication and mention of the grant of the patent: **21.06.2017**
(21) Application number: 15000525.4
(22) Date of filing: 24.02.2015
(51) Int. Cl.: E05C 5/00, E05C 19/14, E05B 7/00, B04B 7/06, E05F 1/10

(54) **Door closing and securing mechanism**
Türschließ- und Sicherungsvorrichtung
Mécanisme de fermeture et de sécurisation de porte

(30) Priority: 26.02.2014 CZ 20140116
(43) Date of publication of application: 16.09.2015
(73) Proprietor: BMT Medical Technology s.r.o., 60200 Brno (CZ)
(72) Inventor: Oplatek, Igor, CZ-60200 Brno (CZ); Trcka, Lubor, CZ-62500 Brno (CZ); Foltýn, Michal, CZ- 61600 Brno (CZ)
(74) Representative: Kendereski, Dusan

(56) References cited:
- DE-U1- 29 812 603
- US-A- 6 079 754
- US-A1- 2012 019 112
- US-B2- 8 403 380

## Description

### Field of the invention

The present invention relates to a door closing and securing mechanism, particularly a mechanism for closing and securing doors of laboratory, medical and sterilization cabinets.

### Background of the invention

The known door locking and securing mechanisms, which constitute the prior art and are typically used in the doors of medical devices, sterilizers or laboratory apparatuses, are usually conceived as linkage-type mechanisms.

A known door closing mechanism is operated by means of a handle that is rigidly coupled with hooks. The axis of rotation of the hooks is identical to that of the handle. The movement of the handle causes the hooks to be driven in rotation. Once the mutual engagement of the hooks and the fixed part of the closing mechanism, which is attached to a cabinet, is achieved, the continuing movement of the handle causes the door to start moving towards the cabinet. The movement of the door, however, is accompanied by friction between the hooks and the fixed part of the closing mechanism. The frictional force is inversely proportional to the distance between the door and the cabinet at any given moment. The above effect hinders the door from being closed or opened in a convenient manner. Moreover, it is often accompanied by undesirable acoustic emissions.

The patent application US2012019112 A1 discloses a latching system for an appliance. The appliance includes a first oven, a first door for the first oven, a second oven and a second door for the second oven. The latching system includes a master latch assembly for locking the first door when it is fully closed; a slave latch assembly for locking the second door when it is fully closed; a cable operatively coupling the two latch assemblies so that a movement of one latch assembly between an unlocking position and a locking position generates a corresponding movement of the other latch assembly between an unlocking position and a locking position; a position switch for one latch assembly; and a controller configured to enable an operation of the first oven and/or the second oven when the position switch detects the latch assembly is in its locking position in conjunction with an operation request.

The patent document US6079754 A discloses a latching assembly, such as for use in assembling and disassembling mating foamed insulating panels, which may have housing components of inexpensive materials such as cellulosic or non-structural plastic materials. A cam shaft passes through an opening in a latch, the cam shaft mounting a cam keeper which has its peripheral surface engaged by a friction clutch (e.g. a cantilevered tongue) which supplies a radial or axial spring force from the exterior of the cam keeper to provide high level of predictable friction force. The cam shaft and a latching pin for cooperation with hook portion of the latch are mounted by metal or structural plastic flanges which engage the inexpensive housing components. An external clutch, such as a spring tab integral with the second metal or structural plastic flange, maintains the position of the cam shaft with respect to the flange unless the cam shaft is positively rotated.

The utility model document DE29812603 U1 discloses a rotating latch lock to secure a movable locking part of a bonnet or a tailboard of a vehicle with a closure hook on the inside of a locking part, operated by a rotational pull movement, the closure hook being operatively connected to a fixed locking element, such as an integrated pin. The closure hook is arranged in a hanging and a limited swinging manner on a turning lever mounted on the locking part and is further rigidly fixed to the centre of said turning lever connected to a rotation handle exerting a rotational pull movement on the closure hook.

The patent document US8403380 B2 discloses a cover closure for housings of laboratory devices and the like, in particular for centrifuge housings, in which a cover hook is engaged by a closure hook and brought into a closing position. The closing and opening movement of the closure hook is essentially caused by an eccentric in connection with a crank guide and a guide path.

### Summary of the invention

The present invention proposes a novel design solution for door closing and securing mechanisms. The design solution according to the invention is simple and enables the particular door to be easily closed and opened.

The above mentioned drawback are largely eliminated by a door closing and securing mechanism, particularly a mechanism for closing and securing doors of laboratory, medical and sterilization cabinets, said mechanism consisting of a fixed part attached to the respective cabinet and a movable part arranged in the door, wherein the movable part comprises at least one plate provided with elastic end stops and inseparably coupled with the handle by means of a center pin with a coaxial end stop attached thereto, the plate being further provided with a pivot eccentrically mounted thereon and carrying at least one pivotally arranged hook, which has a recess for engaging a protrusion of the fixed part, with an opening accommodating one end of a tensile spring and with a compression spring arranged between the plate and the hook.

According to the invention, the door closing and securing mechanism consists of a fixed part and a movable part, one or more of the components of the latter performing an eccentric movement in order to pull the door towards the respective cabinet.

The main advantage of a door, which is provided with the closing and securing mechanism according to the invention, consists in that the opening and closing operation of such door can be considerably facilitated. The door opening and closing operations are carried out through the corresponding movements of the handle requiring just a minimum force to be exerted, the movements of the internal eccentric mechanism being derived from those of the handle.

Furthermore, the technical solution, on which the door closing and securing mechanism according to the present invention is based, enables the force required both for closing the respective cabinet and for opening the same to be kept at a minimum level.

Another advantage of the invention consists in the simple design solution of the closing and securing mechanism.

### Brief description of the drawings

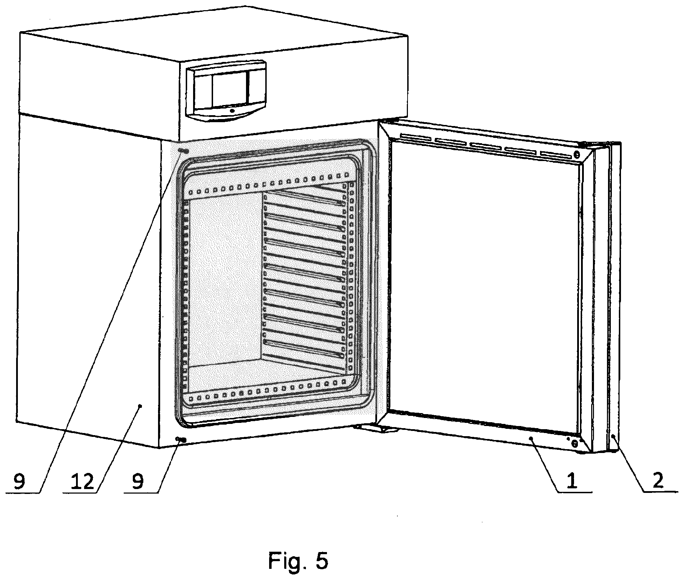

The invention will be further explained with reference to the accompanying drawings in which Fig. 1 shows the door closing and securing mechanism in its open position, Fig. 2 shows the door closing and securing mechanism in the door closing position, Fig. 3 shows the door closing and securing mechanism in its closed position, Fig. 4 shows the movable part of the door closing and securing mechanism, and Fig. 5 shows the placement of the respective components of the door closing and securing mechanism on a cabinet and in a corresponding door.

### Examples of preferred embodiments of the invention

Hereinafter, an exemplary preferred embodiment of the present invention will be described with reference to the corresponding accompanying drawings.

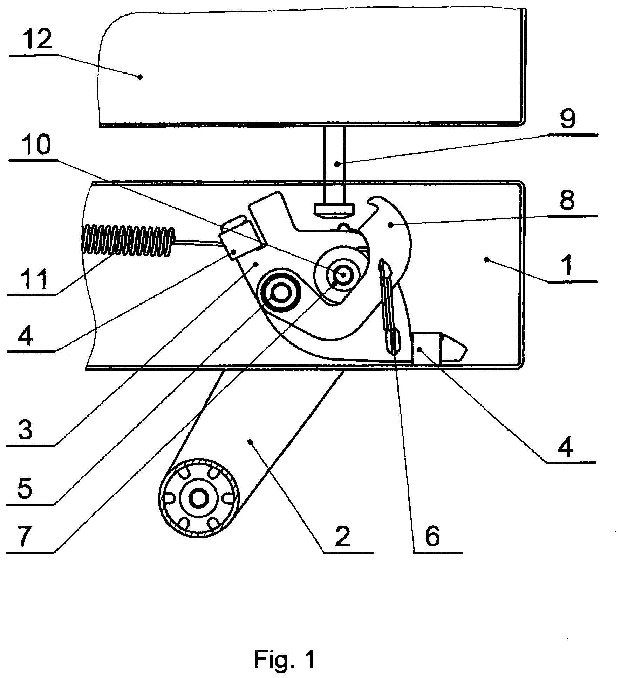

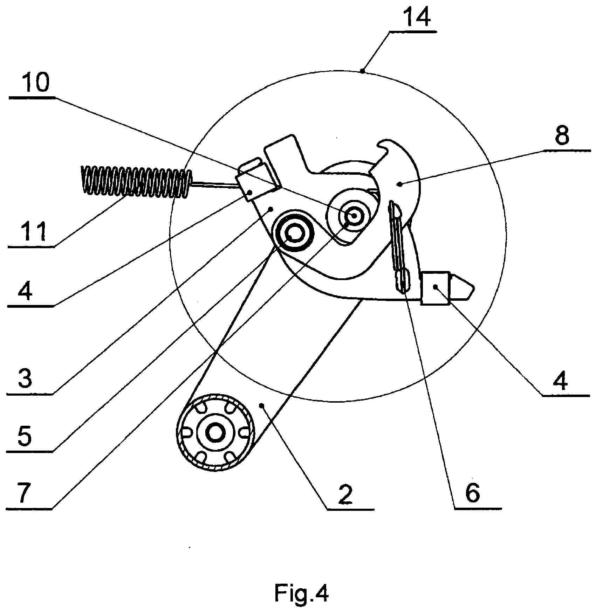

The door closing and securing mechanism according to the present invention consists of the fixed part **9**, which is arranged on the cabinet **12**, and the movable part **14,** which is situated in the door **1** and comprises a plurality of interacting components (**2, 3, 4, 5, 6, 7, 8, 10, 11**), the movement trajectories of the individual components (**3, 4, 5, 6, 7, 8, 10, 11**) being derived from that of the handle **2**. As clearly illustrated in Figs. 1, 2, 3, and 4, the movable part **14** according to the present embodiment of the closing and securing mechanism comprises the plate **3** that is inseparably coupled with the center pin **10** and the handle **2**. The center pin **10** is pivotally interconnected with the door **1**. The end positions of the plate **3** are delimited by the elastic end stops **4**. Furthermore, the plate **3** is provided with the pivot **5** eccentrically mounted thereon and carrying the movable hook **8**. The hook **8** and the plate **3** are interconnected by means of the compression spring **6** for forcing the hook **8** towards the fixed part **9** of the mechanism or towards the coaxial end stop **7**. The end stop **7** is arranged coaxially with respect to the center pin **10** of the plate **3**. The shape of the hook **8** enables the same to engage with the protruding fixed part **9**. Due to the eccentric arrangement of the pivot **5**, the hook **8** approaches to the fixed part **9** during the phase of the closing movement of the door **1** corresponding to the final phase of the movement of the handle **2**.This causes the door **1** to be pulled towards the cabinet **12**. During the phase of the closing movement of the door **1** corresponding to the final phase of the movement of the handle **2,** the hook **8** will rest against the end stop **7** which causes the hook **8** to move away from the fixed part **9** and enables the door **1** to be opened.

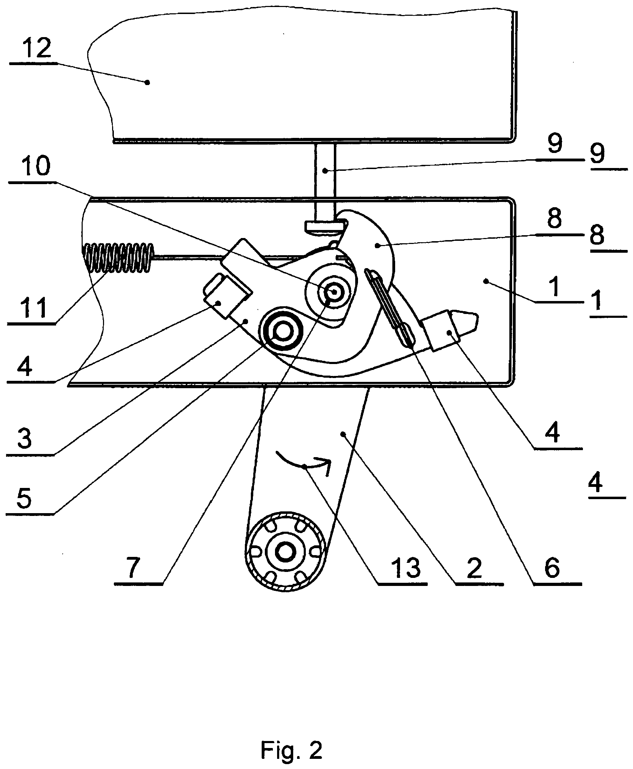

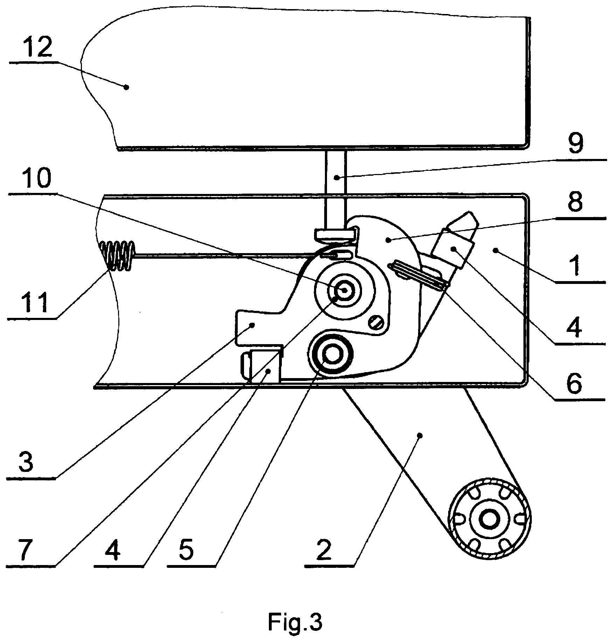

The function of the closing and securing mechanism according to the present embodiment is as follows. In Fig. 1, the closing and securing mechanism is shown in its open position, the hook **8** resting on the end stop **7**. Turning the plate **3** in the direction of the arrow **13** causes the hook **8** to move towards the fixed part **9** mounted on the cabinet **12** and to rest against the same, as illustrated in Fig. 2. Then, the continuing rotational movement of the plate **3** causes the hook **8**, which engages with pro protruding fixed part **9**, to start pulling the door **1** towards the cabinet **12**. If the door assumes its closed position, no moment of force will act on the plate **3**. This means that the position of the plate **3** corresponding to the closed position of the door is maintained by means of the friction produced by the center pin **10** or by means of the action of the tensile spring **11**, as seen in Fig. 3.

### Industrial applicability

The door closing and securing mechanism is usable for operating cabinet doors of laboratory, medical and sterilization apparatuses.

### List of reference numerals

- 1: door
- 2: handle
- 3: plate
- 4: elastic end stop
- 5: pivot
- 6: compression spring
- 7: coaxial end stop
- 8: hook
- 9: fixed part
- 10: center pin
- 11: tension spring
- 12: cabinet
- 13: arrow
- 14: movable part

## Claims

1. Door closing and securing mechanism, particularly a mechanism for closing and securing doors of laboratory, medical and sterilization cabinets, said mechanism consisting of a fixed part (**9**) attached to the respective cabinet (**12**) and a movable part (**14**) arranged in the door (**1**), **characterized in that** the movable part (**14**) comprises at least one plate (**3**) provided with flexible end stops (**4**) and inseparably coupled with the handle (**2**) by means of a center pin (**10**) with a coaxial end stop (**7**) attached thereto, the plate (**3**) being further provided with a pivot (**5**) eccentrically mounted thereon and carrying at least one pivotally arranged hook (**8**), which has a recess for engaging a protrusion of the fixed part (**9**), with an opening accommodating one end of a tensile spring (**11**) and with a compression spring (**6**) arranged between the plate (**3**) and the hook (**8**).

## Patentansprüche

1. Mechanismus zum Türschließen und Türsichern, besonders ein Mechanismus zum Schließen und Sichern von Türen der Labor-, Medizin- und Sterilisationsschränke, wobei das Mechanismus aus einem an dem jeweiligen Schrank (12) befestigten, festen Teil (9) und aus einem in der Tür (1) angeordneten, beweglichen Teil (14) besteht, **dadurch gekennzeichnet, dass** der bewegliche Teil (14) mindestens eine mit flexiblen Endanschlägen (4) versehene und mit einem Griff (2) untrennbar verknüpfte Platte (3), mittels eines Mittelzapfens (10) mit einem daran befestigten koaxialen Endschlag (7), umfasst, wobei die Platte (3) weiter mit einem daran exzentrisch angebrachten Drehpunkt (5) versehen ist und mindestens einen schwenkbar angeordneten Haken (8) trägt, der eine mit einem Vorsprung des festen Teiles (9) einrastende Aussparung aufweist, mit einer Öffnung, die eine Beendigung einer Zugfeder (11) umfasst, und mit einer zwischen der Platte (3) und dem Haken (8) angeordneten Druckfeder (6).

## Revendications

1. Mécanisme de fermeture et de fixation de portes, en particulier un mécanisme de fermeture et de fixation des portes d'armoires de laboratoire, médicales et de stérilisation, ledit mécanisme composé d'une partie (**9**) fixe reliée à l'armoire (**12**) respective et d'une partie (**14**) mobile disposée dans la porte (**1**), **caractérisé en ce que** la partie (**14**) mobile comprend au moins une plaque (**3**) munie de butées (**4**) d'extrémité flexibles et couplée inséparablement à une poignée (**2**) au moyen d'un pivot (**10**) central avec une butée (**7**) d'extremité coaxial attachée sur celui-ci, la plaque (**3**) étant pourvue de plus d'un pivot (**5**) monté excentriquement sur celle-ci et portant au moins un crochet (**8**) disposé de manière pivotante dont la renfoncement embraye la saillie de la partie (**9**) fixe, avec une ouverture logeant une extremité d'un ressort (**11**) de traction et avec un ressort (**6**) de compression disposé entre la plaque (**3**) et le crochet (**8**).
